(19) Europäisches Patentamt / European Patent Office / Office européen des brevets

(11) **EP 3 171 885 B1**

(12) **FASCICULE DE BREVET EUROPEEN**

(45) Date de publication et mention
de la délivrance du brevet:
**27.02.2019 Bulletin 2019/09**

(21) Numéro de dépôt: **15741979.7**

(22) Date de dépôt: **21.07.2015**

(51) Int Cl.:
*A61K 38/01* (2006.01)    *A61K 38/05* (2006.01)
*A61K 35/618* (2015.01)    *A61P 9/12* (2006.01)
*A23L 33/18* (2016.01)

(86) Numéro de dépôt international:
**PCT/EP2015/066652**

(87) Numéro de publication internationale:
**WO 2016/012453 (28.01.2016 Gazette 2016/04)**

(54) **HYDROLYSAT PEPTIDIQUE D'ESCARGOTS, FRACTIONS PEPTIDIQUES, PROCÉDÉ DE FABRICATION, UTILISATION ET COMPOSITIONS POUR INHIBER L'ENZYME DE CONVERSION DE L'ANGIOTENSINE**

PEPTIDHYDROLYSAT VON SCHNECKEN, PEPTIDFRAKTIONEN, HERSTELLUNGSVERFAHREN, VERWENDUNG UND ZUSAMMENSETZUNGEN ZUR HEMMUNG DES ANGIOTENSINUMWANDLUNGSENZYMS

PEPTIDE HYDROLYSATE OF SNAILS, PEPTIDE FRACTIONS, PRODUCTION METHOD, USE AND COMPOSITIONS FOR INHIBITING THE ANGIOTENSIN CONVERTING ENZYME

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **21.07.2014 FR 1457023**

(43) Date de publication de la demande:
**31.05.2017 Bulletin 2017/22**

(73) Titulaire: **Drevet, Pascal
14650 Carpiquet (FR)**

(72) Inventeur: **Drevet, Pascal
14650 Carpiquet (FR)**

(74) Mandataire: **Vidon Brevets & Stratégie
16B, rue de Jouanet
BP 90333
35703 Rennes Cedex 7 (FR)**

(56) Documents cités:
**US-A- 3 885 012**

• KIM Y ET AL: "Melania snails showing biological
activities in Korea", ANNALS OF NUTRITION &
METABOLISM, vol. 58, no. Suppl. 3, 2011, page
105, XP002737331, & 11TH EUROPEAN
NUTRITION CONFERENCE (FENS); MADRID,
SPAIN; OCTOBER 26 -29, 2011
• XIA ET AL: "Characteristics of Bellamya
purificata snail foot protein and enzymatic
hydrolysates", FOOD CHEMISTRY, ELSEVIER
LTD, NL, vol. 101, no. 3, 25 septembre 2006
(2006-09-25), pages 1188-1196, XP005852171,
ISSN: 0308-8146, DOI:
10.1016/J.FOODCHEM.2006.03.031

**Description**

**1. Domaine de l'invention**

Le domaine de l'invention est celui des produits (compléments alimentaires, ingrédients santé, médicaments) destinés à prévenir ou à traiter l'hypertension artérielle, chez l'homme ou l'animal.

Plus précisément, le domaine de l'invention est celui relatif à de tels produits dont le principe actif présente une activité inhibitrice de l'enzyme de conversion de l'angiotensine, ci-après « ECA ».

L'invention concerne ainsi un procédé d'obtention d'un hydrolysat d'escargot permettant d'inhiber l'ECA..

**2. Art antérieur**

La pression artérielle est la pression exercée par le sang sur les parois des vaisseaux sanguins. Un adulte humain présente normalement une pression artérielle inférieure ou égale à 120/80 mmHg. On parle d'hypertension artérielle dès lors que la pression artérielle mesurée dépasse 140/90 mmHg.

L'hypertension artérielle est un facteur de risque majeur, notamment pour le développement d'accidents vasculaires cérébraux (AVC) et des maladies cardio-vasculaires, importantes causes de morbidité et de mortalité en Occident. On estime que 31% de la population adulte aux Etats-Unis souffre d'hypertension et qu'un Américain adulte sur 3 serait en condition de pré-hypertension artérielle. C'est la raison pour laquelle l'hypertension est traitée dès son diagnostic.

En vieillissant, la paroi des vaisseaux sanguins perd de son élasticité et s'épaissit, devenant plus résistante aux déformations liées par l'impulsion du sang. Cette résistance croissante des parois augmente la pression artérielle.

La pression artérielle est également contrôlée par un certain nombre de systèmes métaboliques, y compris la principale voie qu'on appelle le système rénine-angiotensine (SRA). Dans les reins, ce système sécrète une enzyme appelée enzyme de conversion de l'angiotensine (ACE). L'enzyme de conversion de l'angiotensine (ECA) est une exopeptidase permettant de convertir l'angiotensine I en angiotensine II qui est un puissant vasoconstricteur. L'ECA a également le rôle d'inhibiteur de la bradykinine au rôle vasodilatateur. Cette dualité dans son mode d'action fait de l'ECA une cible de choix dans le traitement de l'hypertension artérielle.

Les inhibiteurs de l'ECA permettent donc de réduire la fréquence cardiaque en stimulant la vasodilatation des vaisseaux et par conséquent contribuent à réduire la pression dans le système cardiovasculaire.

Ils sont par conséquent régulièrement prescrits pour le traitement de l'hypertension artérielle, des néphropathies, en particulier des néphropathies diabétiques, des infections aigües du myocarde, des infarctus du myocarde, de l'AVC et leur prévention.

Le principal inhibiteur de l'ECA proposé sur le marché est le captopril (acide (2S)-1-[(2S)-2-methyl-3-sulfanylpropanoyl]-pyrrolidine-2-carboxylique). Le captopril est synthétisé par voie chimique et a été initialement dérivé du venin de serpent jararaca. Il est aujourd'hui le traitement de référence de l'hypertension artérielle, la prévention des récidives de l'infarctus du myocarde et de l'insuffisance cardiaque congestive.

Les effets secondaires de cet inhibiteur de l'ECA sont nombreux : protéinurie, hyperkaliémie, leucopénie, nausées, toux sèches persistantes etc. Outre ces effets indésirables, le captopril provoque des rashs cutanés et des altérations du goût : certains patients se plaignent d'un goût métallique persistant en bouche. D'autres constatent la perte totale de ce sens. Ces derniers effets seraient liés à la présence d'un groupement thiol dans le captopril.

**3. Objectifs de l'invention**

L'invention a notamment pour objectif, dans au moins un mode de réalisation, un procédé permettant de produire une composition d'origine naturelle, alimentaire ou pharmaceutique, permettant d'inhiber l'ECA.

L'invention a également pour objectif de proposer, dans au moins un mode de réalisation, une alternative d'origine naturelle au captopril.

**4. Exposé de l'invention**

Ces objectifs, ainsi que d'autres qui apparaîtront par la suite, sont atteints à l'aide d'un procédé de préparation d'un hydrolysat de tout ou partie de corps d'escargots selon la revendication 1, ledit hydrolysat présentant une activité inhibitrice de l'enzyme de conversion de l'angiotensine.

L'hydrolysat obtenu selon le procédé de l'invention peut être utilisé dans une composition peptidique d'origine naturelle pour inhiber l'ECA, à partir d'escargot. Ainsi, les effets indésirables des médicaments synthétisés par voie chimique sont susceptibles à tout le moins d'être atténués. En particulier, les rashs et les altérations du goût constatées chez les patients traités par le captopril sont susceptibles d'être évités.

Le Déposant a en effet constaté de manière surprenante qu'un hydrolysat peptidique d'escargots permettait d'inhiber l'ECA de manière significative, voire même avec une efficacité comparable à celle du captopril.

Ainsi, le Déposant propose d'utiliser l'hydrolysat peptidique d'escargots afin de fournir une alternative aux inhibiteurs conventionnels d'ECA.

L'origine naturelle de l'hydrolysat permet de limiter les réactions allergiques et les effets indésirables du traitement. Il est effectivement connu que les produits d'origine naturelle entrainent globalement moins d'effets secondaires que les produits synthétisés par voie chimique.

Selon l'invention l'hydrolysat est obtenu à partir d'hépatopancréas d'escargot. Cette partie de l'escargot est particulièrement riche en enzymes et en protéines. Cette variante consiste en pratique à travailler à partir de déchets de l'industrie agro-alimentaire, les hépatopancréas correspondant à la partie de l'escargot non valorisée à titre culinaire. Il est en effet possible de récupérer ces déchets d'escargots pour produire l'hydrolysat selon l'invention. Le coût de production de l'hydrolysat s'en trouve donc réduit.

Avantageusement, l'hydrolysat selon l'invention est obtenu à partir de tout ou partie de corps d'escargots appartenant à au moins une espèce choisie dans le groupe constituée par *Helix aspersa, Helix lucorum, Helix pomatia.* Ces espèces peuvent être soit récoltées par ramassage dans la nature, soit être produites dans un élevage.

De préférence, ledit hydrolysat est obtenu à partir de tout ou partie de corps d'*Helix aspersa.* En effet, l'espèce *Helix aspersa* est une espèce d'escargots d'élevage. La qualité de l'escargot est donc plus facilement maitrisable.

L'hydrolysat obtenu selon le procédé de l'invention comprend préférentiellement une fraction peptidique regroupant les peptides de poids moléculaires allant de 100 Daltons (Da) à 1050 Daltons (Da).

De préférence, la fraction regroupe les peptides de poids moléculaire allant de 100 Da à 350 Da.

Le déposant a en effet constaté que ces fractions présentent une bonne activité inhibitrice de l'ECA.

De manière davantage préférée, la fraction de l'hydrolysat obtenu selon le procédé de l'invention regroupe les peptides de poids moléculaires allant de 100 à 175 Da. Le Déposant a en effet constaté que cette fraction particulière de l'hydrolysat peptidique selon l'invention présente une activité inhibitrice de l'ECA particulièrement intéressante. En effet, l'activité inhibitrice de cette fraction est comparable à celle du captopril. Cette fraction est donc particulièrement intéressante pour la formulation d'une composition, le cas échéant pharmaceutique, d'origine naturelle pour traiter l'hypertension artérielle.

L'hydrolysat obtenu grâce au procédé selon l'invention comprend préférentiellement un mélange de peptides choisi(s) dans le groupe constitué par Arginine-Leucine (RL), Tyrosine-Serine (YS), Tyrosine-Glycine (YG), Tyrosine - Alanine (YA), Leucine-Serine (LS), Leucine-Glycine (LG), Serine-Phénylalanine (SF), Phénylalanine-Glycine (FG), Glycine-Phénylalanine (GF), Acide Aspartique -Phénylalanine (DF), Tyrosine-Méthionine (YM), Valine - Tryptophane (VW), Tyrosine-Phénylalanine (YF), Phénylalanine-Leucine (FL), Phénylalanine- Phénylalanine (FF), Leucine-Acide Aspartique - Alanine (LDA) ou leurs combinaisons, pour prévenir ou traiter l'hypertension artérielle chez l'homme ou l'animal.

Le Déposant a en effet constaté que l'hydrolysat obtenu selon le procédé de l'invention contenai(en)t ces peptides.

De préférence, l'hydrolysat obtenu selon l'invention comprend un mélange de peptides est choisi(s) dans le groupe constitué par Valine - Tryptophane (VW), Tyrosine-Glycine (YG), Tyrosine - Alanine (YA), Serine-Phénylalanine (SF), Phénylalanine-Glycine (FG), Glycine-Phénylalanine (GF), Acide Aspartique -Phénylalanine (DF), Tyrosine-Méthionine (YM) ou leurs combinaisons. Le Déposant a en effet constaté que les fractions peptidiques de l'invention contenant ces peptides étaient celles qui présentaient l'activité inhibitrice de l'ECA la plus intéressante. Par conséquent, le peptide ou mélange de peptide selon l'invention peut être utilisé pour prévenir et/ou traiter l'hypertension artérielle.

L'hydrolysat obtenu grâce au procédé de l'invention peut être intégré dans des compositions pharmaceutiques ou des compléments alimentaires destinés à prévenir ou à réduire l'hypertension, chez l'homme, ou chez l'animal, en particulier le chien ou le chat.

Il peuvt également être incorporé dans des compositions alimentaires fonctionnelles. On entend par « compositions alimentaires fonctionnelles» ou « aliments fonctionnels » des produits alimentaires de grande consommation auquel on ajoute un principe actif qui permet de lutter contre et/ou de prévenir une pathologie, ou de diminuer un facteur de risque pour une pathologie. Par exemple, on peut citer les margarines enrichies en oméga 3, 6 et 9 qui permettent de lutter contre l'excès de cholestérol (qui n'est pas une maladie en soi mais est un facteur de risque pour les plaques d'athérome et les pathologies cardiovasculaires). A titre de précision, lorsque ledit principe actif est intégré dans une composition alimentaire pour en faire une composition alimentaire fonctionnelle, ou aliment fonctionnel, il peut être désigné sous le terme « ingrédient santé ».

Une approche holistique du traitement de l'hypertension artérielle permet de diminuer la dose de médicament à prendre pour le patient. Cela permet également de traiter une hypertension artérielle légère.

L'invention couvre donc également toute composition comprenant un hydrolysat obtenu par le procédé selon l'invention , dans un complément alimentaire, à usage humain ou vétérinaire, pour son utilisation pour d prévenir l'hypertension artérielle.

De tels compléments alimentaires peuvent être formulés par exemple sous forme de gélules, de comprimés ou de capsules molles contenant un liquide. Ces compléments alimentaires peuvent être destinés à des individus, humains ou animaux, présentant une prédisposition à l'hypertension artérielle. C'est notamment le cas des individus âgés dont les parois des vaisseaux perdent naturellement de leur élasticité. C'est également le cas d'individus dont l'hypertension artérielle est légèrement trop élevée et qu'il est nécessaire de surveiller. Dans ce dernier cas, ces individus pourraient être intéressés par un complément alimentaire permettant de maintenir leur pression artérielle dans les normes.

De tels compléments alimentaires peuvent contenir au moins un excipient tel un agent de charge, un lubrifiant, un agent d'enrobage, un conservateur, un stabilisant, un régulateur de pH etc.

Ainsi, l'invention propose une alternative aux traitements conventionnels, et notamment au captopril. L'absence de groupement thiol dans les produits selon l'invention est susceptible de supprimer les effets indésirables précédemment cités comme l'altération du goût et les rashs cutanés.

Le médicament contenant l'hydrolysat obtenu selon l'invention peut contenir au moins un excipient tel un agent de charge, un lubrifiant, un agent d'enrobage, un conservateur, un stabilisant, un régulateur de pH etc.

Selon la revendication 1, l'invention concerne un procédé de préparation d'un hydrolysat selon l'invention comprenant les étapes de :

a) broyage de tout ou partie de corps d'escargots pour obtenir un broyât;
b) mélange dudit broyat avec de l'eau purifiée ;
c) hydrolyse par alcalase du mélange obtenu à l'étape b) pour obtenir un hydrolysat;
d) séparation liquide-solide de l'hydrolysat obtenu à l'étape c) pour obtenir une phase liquide et une phase solide;
e) séchage de la phase liquide obtenue à l'étape d).

Avantageusement, ladite étape c) d'hydrolyse est effectuée à une température de 55°C., en pratique en utilisant, pour l'étape b), de l'eau chauffée à cette température. Cette température permet d'activer les protéases et de lyser les protéines contenues dans les escargots afin d'obtenir un hydrolysat selon l'invention.

Dans une variante intéressante, le procédé selon l'invention comprend en outre une étape de concentration la phase liquide obtenue à l'étape d) entre l'étape de séparation liquide-solide d) et l'étape de séchage e).

L'étape de concentration permet notamment d'accélérer le séchage de ladite phase liquide. Cette étape optionnelle de concentration peut se faire par évaporation sous vide.

Le procédé selon l'invention peut également comprendre une étape supplémentaire d'ultrafiltration mise en oeuvre après l'étape de séparation liquide-solide et avant l'étape de concentration. Une telle étape d'ultrafiltration permet d'éliminer les éléments de petite taille que la séparation liquide-solide n'aurait pas réussi à ôter. Avantageusement, la technique de filtration mise en oeuvre est une technique d'ultrafiltration tangentielle sur membrane présentant un seuil de coupure de 10 000 Daltons (Da). Ceci signifie que la membrane retient les particules dont le poids moléculaire est supérieur ou égal à 10 000 Da. La technique de filtration tangentielle consiste à faire passer le fluide tangentiellement à la surface du filtre. C'est la pression du fluide qui lui permet de traverser le filtre. Les particules, dans ce cas, restent dans le flux de circulation tangentiel, ce qui permet de ralentir le colmatage de la membrane de filtration.

### 5. Liste des figures

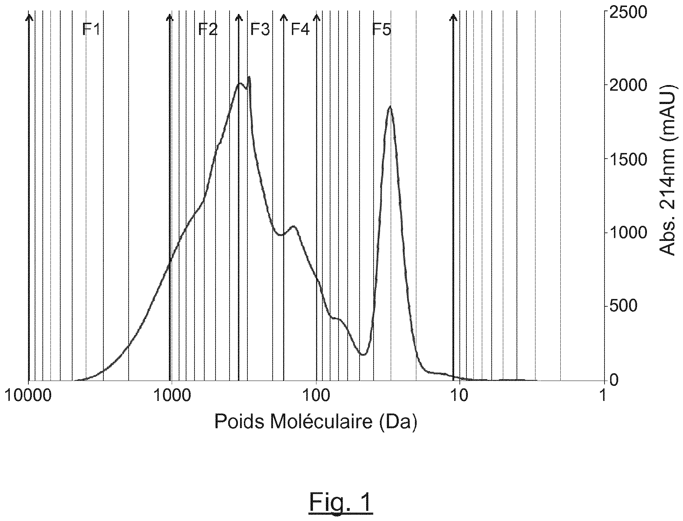

D'autres caractéristiques et avantages de l'invention apparaîtront plus clairement à la lecture de la description suivante d'un mode de réalisation préférentiel, donné à titre de simple exemple illustratif et non limitatif, et de la figure 1 annexée. La figure 1 présente un chromatogramme obtenu après chromatographie d'exclusion stérique d'un hydrolysat selon l'invention.

### 6. Description d'un mode de réalisation de l'invention

Le principe général de l'invention repose sur la proposition d'une alternative naturelle aux inhibiteurs conventionnels de l'ECA obtenus par synthèse chimique. L'alternative naturelle proposée par le Déposant consiste en un hydrolysat, ses fractions peptidiques et/ou les peptides isolés de cet hydrolysat pour inhiber l'ECA. De plus, l'invention propose également d'utiliser les propriétés inhibitrices d'un tel hydrolysat, obtenu à partir d'escargots, de ses fractions peptidiques et/ou de peptides le constituant pour proposer des médicaments, des compléments alimentaires et des aliments fonctionnels permettant de prévenir et/ou traiter l'hypertension artérielle.

6.1. Préparation d'un hydrolysat selon le procédé de l'invention.

On prépare un hydrolysat selon le procédé de l'invention en utilisant environ 250 kg d'escargots choisis parmi les espèces *Helix aspersa, Helix lucorum* et *Helix pomatia* ou de l'hépatopancréas d' *Helix aspersa, Helix lucorum* et *Helix pomatia.* Il est possible d'utiliser des lots d'escargots ne contenant qu'un espèce ou un mélange de deux ou trois des espèces citées. De préférence, on utilise *Helix aspersa* car ce sont des escargots d'élevage. La qualité du lot est donc plus facile à maitriser. L'hydrolysat peut être préparé à partir d'escargots congelés. En ce cas, les escargots sont mis à décongeler en chambre froide afin que la décongélation soit lente. Une décongélation lente permet de préserver les enzymes et protéines de l'escargot.

Une fois décongelés, les escargots sont broyés selon toute technique connue de l'homme du métier. Un volume de 250 L d'eau purifiée est chauffé à 55°C avant d'être mélangé aux escargots broyés.

Afin de procéder à l'hydrolyse enzymatique, on ajoute au mélange escargots broyés - eau à 55°C une quantité de 1% massique d'alcalase par rapport au poids des escargots, soit 2,5 kg de protéase. Le mélange est maintenu à 55°C, pH 8, pendant 2 heures pour que la réaction enzymatique se fasse. L'hydrolyse est ensuite arrêtée au bout de 2 heures en chauffant le mélange à 90°C pendant 10 secondes, afin d'inactiver la protéase. On obtient ainsi un mélange hydrolysé.

Le mélange hydrolysé est ensuite soumis à une étape de centrifugation à 4000 rpm, pendant 5 minutes, à température ambiante (centrifugeuse KR25i, JOUAN). Le surnageant est récupéré et le culot comprenant les débris non digérés des corps d'escargots est éliminé.

Une filtration optionnelle est mise en oeuvre pour éliminer les débris sur une membrane présentant un seuil de coupure de 10 000 Da . A ce stade, on obtient un surnageant clair.

De préférence, le surnageant est ensuite soumis à une étape de concentration par évaporation sous vide afin d'éliminer l'eau superflue et de concentrer les enzymes dans le surnageant. Plus précisément, le surnageant est injecté soit dans un évaporateur rotatif sous vide, soit dans un évaporateur sous vide à colonne. L'évaporation se fait ainsi pendant une durée comprise entre 4 et 6 heures, à une température finement contrôlée comprise entre 40°C et 45°C. Ainsi, l'étape de séchage est facilitée. Cette plage de température permet de sécher le surnageant sans endommager les peptides contenus dans l'hydrolysat.

Enfin, le surnageant, éventuellement concentré, est soumis à une étape de séchage. De préférence, on procède à un séchage par flux d'air dont la température ne dépasse pas 45°C. Ainsi, la qualité des peptides et leur activité sont préservées. D'autres techniques peuvent être mises en oeuvre pour sécher l'hydrolysat et notamment l'atomisation et la lyophilisation. Le choix parmi ces techniques est fait en fonction des contraintes industrielles et de l'utilisation de l'hydrolysat.

## 6.2. Préparation de fractions peptidiques

Des fractions peptidiques de l'hydrolysat sont ensuite obtenues en soumettant l'hydrolysat obtenu selon le procédé décrit au point 6.1 à une chromatographique d'exclusion stérique sur colonnes Superdex Peptides HT 10/300 (GE Healthcare) dont la zone de fractionnement de gel est comprise entre 100 Da et 7000 Da.

La phase stationnaire est composée de billes poreuses d'agarose et de dextran d'un diamètre moyen de 13 $\mu$m. La phase mobile est composée d'un mélange d'eau, d'acétonitrile et de trifluoroacétate dans les proportions volumiques respectives (70/30/0,1). L'absorbance est mesurée à 214 nm et est enregistré par l'automate à une fréquence de 0,3 Hz.

La figure 1 représente le chromatogramme obtenu. On distingue 5 fractions ayant respectivement des poids moléculaires apparent compris entre :

- fraction 1 : de 1050 Da à 10000 Da ;
- fraction 2 : de 350 Da à 1050 Da ;
- fraction 3 : de 175 Da à 350 Da ;
- fraction 4 : de 100 Da à 175 Da ; et
- fraction 5 : de 0 à 100 Da.

## 6.3. Préparation de peptides isolés

L'isolation des peptides à partir de chacune des fractions est réalisée par chromatographie en phase liquide à haute pression (HPLC) suivie d'une spectrométrie de masse (MS) selon toute méthode bien connue de l'homme de l'art.

L'HPLC permet de fractionner les molécules en fonction de leur hydrophobie. La sortie de la colonne d'HPLC est connectée à un spectromètre de masse de type triple quadripole. Ce type de spectromètre permet de combiner deux analyseurs quadripolaires en série et de réaliser des MS en tandem, ou MS[2]. L'analyse MS[2] permet de déterminer la masse et la structure des peptides en une seule injection.

La fragmentation des fractions produites au point 6.2 a permis d'isoler 17 peptides. Les résultats de la spectrométrie de masse sont présentés dans le tableau ci-dessous :

Tableau 1

| Temps de rétention | Rapport masse sur charge m/z | Séquence peptidique | Pourcentage |
|---|---|---|---|
| 5,32 | 288,43 | RL | 2 |
| 6,61 | 269,39 | YS | 4 |
| 6,89 | 239,23 | YG | 4 |
| 6,89 | 253,3 | YA | 3 |
| 7,74 | 166 | F | 1 |
| 7,74 | 219,21 | LS | 4 |
| 9,57 | 189,33 | LG | 3 |
| 11,57 | 281,22 | VY | 2 |
| 12,35 | 253,3 | SF | 6 |
| 13,06 | 223,27 | FG ou GF | 5 |
| 13,94 | 281,3 | DF | 1 |
| 13,94 | 313,28 | YM | 1 |
| 19,75 | 304,31 | VW | 4 |
| 19,75 | 329,5 | YF | 2 |
| 22,52 | 279,48 | FL | 2 |
| 24,11 | 313,48 | FF | 2 |
| 24,29 | 318,38 | LDA | 2 |

6.4. Détermination de l'activité inhibitrice de l'hydrolysat, des fractions et des peptides.

On mesure le potentiel inhibiteur de l'hydrolysat total, des 5 fractions isolées et des peptides obtenus par fragmentation des fractions grâce à la méthode décrite par Cushman et Cheung (Cushman DW et al. , Spectrophotometric assay and properties of the angiotensin-converting enzyme of rabbit lung ; Biochem Pharmacol. 1971 Jul;20(7):1637-48).

Brièvement, cette méthode repose sur l'hydrolyse du substrat Hippuryl-L-Histidyl-L-Leucine (HHL) par l'ECA pour produire de l'acide hippurique (HA) et de l'Histydil-Leucine. Après incubation à 37°C pendant 30 minutes dans du tampon borate de sodium pH 8,3, la réaction est arrêtée par addition de HCl. La séparation du substrat HHL et du produit HA est réalisé par chromatographie liquide haute pression en phase inversée (RP-HPLC) sur une colonne C18 (Kinetex). L'élution se fait par un mélange d'eau (75% v/v) et d'acétonitrile (25% V/V). La quantification se fait par une détection à 228 nm et l'aire sous courbe (ASC) de chaque pic est mesurée.

La détermination de l'$IC_{50}$ de chaque peptide se fait en utilisant différentes concentrations de peptide, pour une concentration constante de HHL fixée à 5 mM. La comparaison entre la concentration de HA produite pour chaque concentration d'échantillon avec celle obtenue sans addition de peptide permet la détermination de l'$IC_{50}$.

A titre de témoin positif, le captopril est également testé en parallèle de l'hydrolysat, des fractions et des peptides. Le témoin négatif est le substrat HHL seul, sans ECA, dilué dans du tampon borate pH 8,3. L'échantillon est le peptide, la fraction ou l'hydrolysat total à tester.

L'indice $IC_{50}$ correspond à la concentration d'inhibiteur nécessaire pour inhiber 50% de l'ECA dans le test : plus la concentration d'inhibiteur nécessaire pour inhiber l'ECA est faible, plus l'$IC_{50}$ est faible et plus l'inhibiteur est puissant.

Le pourcentage d'inhibition de l'ECA est calculé selon la formule suivante :

$$\textbf{Pourcentage d'inhibition ECA } (\%) = \frac{ASC_{HA}(Captopril) - ASC_{HA}(Echantillon)}{ASC_{HA}(Captopril) - ASC_{HA}(HHL)} \times 100$$

Les résultats des mesures du potentiel inhibiteur sont présentés dans le tableau ci-dessous.

Tableau 2

| Echantillon | $IC_{50}$ ($\mu$g/ml) |
|---|---|
| Captopril | 0,005 |
| Hydrolysat total | 56,8 |
| Fraction 1 | / |
| Fraction 2 | 127,9 |
| Fraction 3 | 15,9 |
| Fraction 4 | 0,007 |
| Fraction 5 | / |
| YG | 72,1 |
| VY | 11,3 |
| SF | 130,2 |
| FG ou GF | 279 |
| DF | 360 |
| VW | 1,6 |

Comme on peut l'observer au vu des résultats présentés au tableau 2, la fraction 4, de poids moléculaire apparent de 100 Da à 175 Da présente un potentiel d'inhibition de l'ECA très proche de celui du captopril. Les fractions 2, 3 ou 4, de préférence la fraction 4, peuvent donc être avantageusement utilisées comme médicament destiné à traiter et/ou prévenir l'hypertension artérielle.

On constate également que les dipeptides VY (Valine-Tyrosine) et VW (Valine - Tryptophane) possèdent des $IC_{50}$ faibles. Ces peptides peuvent donc être avantageusement incorporés à un produit alimentaire pour en faire un aliment fonctionnel destiné à prévenir ou réduire l'hypertension artérielle. Ils peuvent également être incorporés dans une composition pharmaceutique ou un complément alimentaire, pour la prévention et/ou le traitement de l'hypertension artérielle.

L'hydrolysat total présente également une valeur d'$IC_{50}$ intéressante pour l'ECA. De la même manière, l'hydrolysat peut être utilisé comme médicament ou être incorporé dans un aliment fonctionnel ou un complément alimentaire pour traiter, réduire ou prévenir l'hypertension artérielle.

L'hydrolysat, les fractions peptides qui le composent et les peptides qui en ont été isolés permettent d'éliminer, ou à tout le moins de réduire, les effets indésirables induits par les traitements actuels de l'hypertension.

**Revendications**

1. Procédé de préparation d'un hydrolysat peptidique présentant une activité inhibitrice de l'enzyme de conversion de l'angiotensine, comprenant les étapes de:

    a) broyage d'hépatopancréas d'escargots pour obtenir un broyat ;
    b) mélange dudit broyat avec de l'eau purifiée ;
    c) hydrolyse enzymatique par alcalase du mélange obtenu à l'étape b) pour obtenir un hydrolysat ;
    d) séparation liquide-solide de l'hydrolysat obtenu à l'étape c) pour obtenir une phase liquide et une phase solide ;
    e) séchage de la phase liquide obtenue à l'étape d).

2. Procédé selon la revendication 1 **caractérisé en ce qu'**il comprend une étape d'ultrafiltration mise en oeuvre après l'étape d) de séparation liquide-solide.

3. Procédé selon la revendication 1 ou 2 comprenant en outre une étape de concentration de la phase liquide effectuée avant l'étape e) de séchage.

4. Procédé selon l'une quelconque des revendications 1 à 3 **caractérisé en ce que** lesdits escargots appartiennent à au moins une espèce choisie dans le groupe constitué par *Helix aspersa, Helix lucorum, Helix pomatia*.

5. Procédé selon la revendication 4 **caractérisé en ce que** lesdits escargots sont de l'espèce *Helix aspersa*.

6. Procédé selon l'une quelconque des revendications 1 à 5 **caractérisé en ce que** ledit hydrolysat comprend un mélange de peptides choisi(s) dans le groupe constitué par Arginine-Leucine (RL), Tyrosine-Serine (YS), Tyrosine-Glycine (YG), Tyrosine - Alanine (YA), Leucine-Serine (LS), Leucine-Glycine (LG), Serine-Phénylalanine (SF), Phénylalanine-Glycine (FG), Glycine-Phénylalanine (GF), Acide Aspartique-Phénylalanine (DF), Tyrosine-Méthionine (YM), Valine-Tryptophane (VW), Tyrosine-Phénylalanine (YF), Phénylalanine-Leucine (FL), Phénylalanine-Phénylalanine (FF), Leucine-Acide Aspartique - Alanine (LDA), Valine-Tyrosine (VY).

7. Procédé selon la revendication 6 **caractérisé en ce que** ledit hydrolysat comprend un mélange de peptides choisi(s) dans le groupe constitué par Valine - Tryptophane (VW), Tyrosine-Glycine (YG), Serine-Phénylalanine (SF), Glycine-Phénylalanine (GF), Acide Aspartique -Phénylalanine (DF), Valine-Tyrosine (VY).

8. Procédé selon l'une quelconque des revendications 6 ou 7 **caractérisé en ce que** ledit mélange de peptides regroupe des peptides de poids moléculaires allant de 350 Da à 1050 Da.

9. Procédé selon la revendication 6 ou 7 **caractérisé en ce que** ledit mélange de peptides regroupe des peptides de poids moléculaire allant de 175 Da à 350 Da.

10. Procédé selon la revendication 6 ou 7 **caractérisé en ce que** ledit mélange de peptides regroupe des peptides de poids moléculaire allant de 100 Da à 175 Da.

11. Hydrolysat obtenu par le procédé selon l'une des revendications 1 à 10 pour son utilisation, dans une composition alimentaire fonctionnelle à usage humain ou animal, pour la prévention de l'hypertension artérielle.

**Patentansprüche**

1. Verfahren zur Herstellung eines Peptidhydrolysats mit einer Angiotensinumwandlungs-Enzym-Inhibitoraktivität, umfassend die folgenden Schritte:

   a) Mahlen des Hepatopankreas von Schnecken, um ein gemahlenes Materials zu erhalten;
   b) Mischen des gemahlenen Materials mit gereinigtem Wasser;
   c) enzymatische Hydrolyse der in Schritt b) erhaltenen Mischung durch Alkalase, um ein Hydrolysat zu erhalten;
   d) Flüssig-Fest-Trennung des in Schritt c) erhaltenen Hydrolysats, um eine flüssige Phase und eine feste Phase zu erhalten;
   e) Trocknung der in Schritt d) erhaltenen flüssigen Phase.

2. Verfahren nach Anspruch 1, **dadurch gekennzeichnet, dass** es einen Ultrafiltrationsschritt umfasst, der nach dem Schritt d) der Flüssig-Fest-Trennung durchgeführt wird.

3. Verfahren nach Anspruch 1 oder 2, ferner umfassend einen Schritt des Konzentrierens der flüssigen Phase, der vor dem Trocknungsschritt e) ausgeführt wird.

4. Verfahren nach einem beliebigen der Ansprüche 1 bis 3, **dadurch gekennzeichnet, dass** die Schnecken zu mindestens einer Spezies gehören, ausgewählt aus der Gruppe bestehend aus *Helix aspersa, Helix lucorum, Helix pomatia*.

5. Verfahren nach Anspruch 4, **dadurch gekennzeichnet, dass** die Schnecken von der Spezies *Helix aspersa* sind.

6. Verfahren nach einem beliebigen der Ansprüche 1 bis 5, **dadurch gekennzeichnet, dass** das Hydrolysat eine Mischung von Peptiden umfasst, ausgewählt aus der Gruppe bestehend aus Arginin-Leucin (RL), Tyrosin-Serin (YS), Tyrosin-Glycin (YG), Tyrosin-Alanin (YA), Leucin-Serin (LS), Leucin-Glycin (LG), Serin-Phenylalanin (SF), Phenylalanin-Glycin (FG), Glycin-Phenylalanin (GF), Asparaginsäure-Phenylalanin (DF), Tyrosin-Methionin (YM), Valin-Tryptophan (VW), Tyrosin-Phenylalanin (YF), Phenylalanin-Leucin (FL), Phenylalanin-Phenylalanin (FF), Leucin-Asparaginsäure-Alanin (LDA), Valin-Tyrosin (VY).

**7.** Verfahren nach Anspruch 6, **dadurch gekennzeichnet, dass** das Hydrolysat eine Mischung von Peptiden umfasst, ausgewählt aus der Gruppe bestehend aus Valin-Tryptophan (VW), Tyrosin-Glycin (YG), Serin-Phenylalanin (SF), Glycin-Phenylalanin (GF), Asparaginsäure-Phenylalanin (DF), Valin-Tyrosin (VY).

**8.** Verfahren nach einem beliebigen der Ansprüche 6 oder 7, **dadurch gekennzeichnet, dass** die Mischung von Peptiden Peptide mit einem Molekulargewicht im Bereich von 350 Da bis 1050 Da umfasst.

**9.** Verfahren nach Anspruch 6 oder 7, **dadurch gekennzeichnet, dass** die Mischung von Peptiden Peptide mit einem Molekulargewicht im Bereich von 175 Da bis 350 Da umfasst.

**10.** Verfahren nach Anspruch 6 oder 7, **dadurch gekennzeichnet, dass** die Mischung von Peptiden Peptide mit einem Molekulargewicht im Bereich von 100 Da bis 175 Da umfasst.

**11.** Hydrolysat, das durch das Verfahren nach einem der Ansprüche 1 bis 10 erhalten wird, zur Verwendung in einer funktionellen Nahrungsmittelzusammensetzung für die Verwendung bei Menschen oder Tieren zur Vorbeugung von arterieller Hypertonie.

**Claims**

**1.** Method for preparing a peptide hydrolysate having an inhibiting activity of the angiotensin conversion enzyme, comprising the steps of:

a) grinding snail hepatopancreases to obtain ground particles;
b) mixing said ground particles with purified water;
c) enzymatic hydrolysing by alkalising the mixture obtained in step b) to obtain a hydrolysate;
d) separating of liquid and solid from the hydrolysate obtained in step c) to obtain a liquid phase and a solid phase;
e) drying the liquid phase obtained in step d).

**2.** Method according to claim 1, **characterised in that** it comprises an ultrafiltration step carried out after step d) of separating liquid and solid.

**3.** Method according to claim 1 or 2 further comprising a step of concentrating the liquid phase carried out before the drying step e).

**4.** Method according to any one of claims 1 to 3, **characterised in that** said snails belong to at least one species selected from the group made up of *Helix aspersa, Helix lucorum, Helix pomatia.*

**5.** Method according to claim 4, **characterised in that** said snails are of the species *Helix aspersa.*

**6.** Method according to any one of claims 1 to 5, **characterised in that** said hydrolysate comprises a mixture of peptides selected from the group made up of Arginine-Leucine (RL), Tyrosine-Serine (YS), Tyrosine-Glycine (YG), Tyrosine-Alanine (YA), Leucine-Serine (LS), Leucine-Glycine (LG), Serine-Phenylalanine (SF), Phenylalanine-Glycine (FG), Glycine-Phenylalanine (GF), Aspartic Acid-Phenylalanine (DF), Tyrosine-Methionine (YM), Valine-Tryptophan (VW), Tyrosine-Phenylalanine (YF), Phenylalanine-Leucine (FL), Phenylalanine-Phenylalanine (FF), Leucine-Aspartic Acid-Alanine (LDA), Valine-Tyrosine (VY).

**7.** Method according to claim 6, **characterised in that** said hydrolysate comprises a mixture of peptides selected from the group made up of Valine-Tryptophan (VW), Tyrosine-Glycine (YG), Serine-Phenylalanine (SF), Glycine-Phenylalanine (GF), Aspartic Acid-Phenylalanine (DF), Valine-Tyrosine (VY).

**8.** Method according to any one of claims 6 or 7, **characterised in that** said mixture of peptides groups together peptides of molecular weights ranging from 350 Da to 1050 Da.

**9.** Method according to claim 6 or 7, **characterised in that** said mixture of peptides groups together peptides of molecular weight ranging from 175 Da to 350 Da.

**10.** Method according to claim 6 or 7, **characterised in that** said mixture of peptides groups together peptides of molecular weight ranging from 100 Da to 175 Da.

11. Hydrolysate obtained by the method according to any one of claims 1 to 10 for the use thereof, in a functional food composition for human or animal use, to prevent arterial hypertension.

Fig. 1

## RÉFÉRENCES CITÉES DANS LA DESCRIPTION

**Littérature non-brevet citée dans la description**

- **CUSHMAN DW et al.** Spectrophotometric assay and properties of the angiotensin-converting enzyme of rabbit lung. *Biochem Pharmacol.,* Juillet 1971, vol. 20 (7), 1637-48